# FASCICULE DE BREVET EUROPEEN

(11) **EP 2 945 509 B1**
(45) Date de publication et mention de la délivrance du brevet: **16.10.2019**
(21) Numéro de dépôt: 14703137.1
(22) Date de dépôt: 13.01.2014
(51) Int. Cl.: A45D 24/00

(54) **SYSTEME INFORMATIF UTILISANT UNE BROSSE A CHEVEUX INSTRUMENTEE ET CONNECTEE**
INFORMATIONSSYSTEM UNTER VERWENDUNG EINER INSTRUMENTIERTEN UND VERBUNDENEN HAARBÜRSTE
INFORMATIVE SYSTEM USING AN INSTRUMENTED AND CONNECTED HAIRBRUSH

(30) Priorité: 15.01.2013 FR 1350345
(43) Date de publication de la demande: 25.11.2015
(73) Titulaire: Withings, 92130 Issy-les-Moulineaux (FR)
(72) Inventeur: HUTCHINGS, Cédric, 92130 Issy Les Moulineaux (FR); CARREEL, Eric, 92190 Meudon (FR)
(74) Mandataire: Cabinet Plasseraud
(86) Numéro de dépôt international: PCT/FR2014/050051
(87) Numéro de publication internationale: WO 2014/111646

(56) Documents cités:
- EP-A1- 1 297 781
- FR-A1- 2 964 023
- US-A- 4 167 869
- US-A1- 2008 102 953
- US-A1- 2009 147 081

## Description

La présente invention est relative aux ustensiles de coiffage, de type peigne ou brosse à cheveux, et aux systèmes incorporant de tels ustensiles de coiffage.

Le document US 2008/102953 divulgue une brosse en relation avec un appareil de type console de jeu.

Plus particulièrement, l'invention concerne un ustensile de coiffage ayant un corps et un manche, utilisé par un utilisateur ou une utilisatrice pour se coiffer ou plus généralement pour entretenir sa chevelure.

Lorsque l'ustensile parcourt une portion de chevelure, il est soumis à des efforts engendrés par la résistance de la chevelure engagée dans les poils, dents ou picots du corps de l'ustensile. Ces efforts sont variables et sont représentatifs d'une certaine façon de la bonne santé ou de certains défauts de ladite chevelure. Cependant, il est très difficile pour un utilisateur d'interpréter ces efforts ou les variations de ces efforts.

Il est donc apparu un besoin de proposer une solution pour permettre une meilleure interprétation des efforts et mouvements mis en jeu au cours des actions de coiffage réalisées par un utilisateur.

A cet effet, l'invention propose un système selon la revendication 1 et un procédé selon la revendication 8.

En outre, l'utilisateur peut prendre connaissance de certaines de ces données acquises par le capteur sur un écran d'affichage d'un appareil mobile par exemple de type Smartphone ou dans un browser web dans un autre exemple.

Dans des modes de réalisation selon l'invention, on peut éventuellement avoir recours en outre à l'une et/ou à l'autre des dispositions suivantes :
- l'ustensile de coiffage peut comprendre en outre au moins une caméra et/ou un capteur optique, adapté pour acquérir des images (notamment des cheveux ou du cuir chevelu), l'unité électronique étant configurée pour mettre en forme et transmettre ces images ; moyennant quoi ces images peuvent être transmises en complément à l'entité distante pour obtenir une analyse encore plus complète;
- l'ustensile de coiffage peut comprendre en outre au moins un microphone, adapté pour mesurer des frottements engendrés par le mouvement de coiffage, l'unité électronique étant configurée pour mettre en forme et transmettre des données correspondantes ; moyennant quoi l'analyse des vibrations peut être utilisée pour affiner l'analyse des données concernant la chevelure ;
- l'ustensile de coiffage peut comprendre en outre au moins un capteur de température et/ou un capteur d'humidité, pour acquérir les caractéristiques courantes d'environnement atmosphérique ; moyennant quoi les conditions environnementales sont utilisées pour affiner l'analyse des données concernant la chevelure ;
- l'ustensile de coiffage peut comprendre en outre au moins un espace mémoire adapté pour permettre le stockage des données collectées pendant le ou les mouvements de coiffage, préalablement à une transmission ultérieure vers l'entité distante ; de sorte qu'on peut minimiser l'utilisation de la liaison sans fil, et en outre, les données ne sont pas perdues en cas d'indisponibilité momentanée de la connexion sans fil ;

L'invention vise également un **système** informatif comprenant un ustensile de coiffage tel que décrit précédemment, et une entité distante formée par un appareil mobile de type Smartphone pouvant être relié par une connexion sans fil audit ustensile de coiffage et configuré pour afficher des données transmises par l'ustensile de coiffage.

De plus, l'entité distante peut être formée par un appareil mobile, par exemple un Smartphone, configuré pour afficher certaines des données transmises par l'ustensile de coiffage et donner des indications à l'utilisateur à propos de sa chevelure soumise à coiffure.

De plus, l'entité distante peut comprendre à la fois un appareil mobile et un serveur informatique pouvant être connecté à l'appareil mobile, afin d'échanger des données de synthèse relatives au coiffage.

L'invention vise également un **procédé** mis en oeuvre dans un système informatif tel que décrit précédemment, le procédé comprenant les étapes :
**a**- acquérir des informations relatives au moins aux mouvements de coiffage d'un utilisateur,
**b**- transmettre des données correspondantes aux dites informations vers une entité distante formée par un appareil mobile de type Smartphone,
**c**- afficher lesdites données correspondantes sur un écran de l'entité distante,
f**-** fournir en retour à l'utilisateur de l'ustensile des indications à propos de la chevelure soumise à coiffure.

De plus, l'entité distante peut être formée par un appareil mobile, les indications à propos de la chevelure soumise à coiffure étant données à l'utilisateur par un message électronique sur l'appareil mobile.

En outre, le procédé peut comprendre optionnellement les étapes :
**d**- transmettre des données de synthèse relatives au coiffage vers le serveur informatique,
**e**- recevoir du serveur en retour des préconisations de soins ou de produits à appliquer à la chevelure considérée et les afficher sur l'appareil mobile.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure 1 est une vue générale en perspective d'un ustensile de coiffage selon l'invention,
- la figure 2 est une vue générale en perspective de l'ustensile de coiffage de la figure 1 en cours d'utilisation par un utilisateur,
- la figure 3 montre de façon schématique un schéma de principe de l'ustensile de coiffage de la figure 1, dans un système informatif dont il fait partie,
- la figure 4 montre de façon schématique une coupe longitudinale de l'ustensile de coiffage de la figure 1.

Sur les différentes figures, les mêmes références désignent des éléments identiques ou similaires.

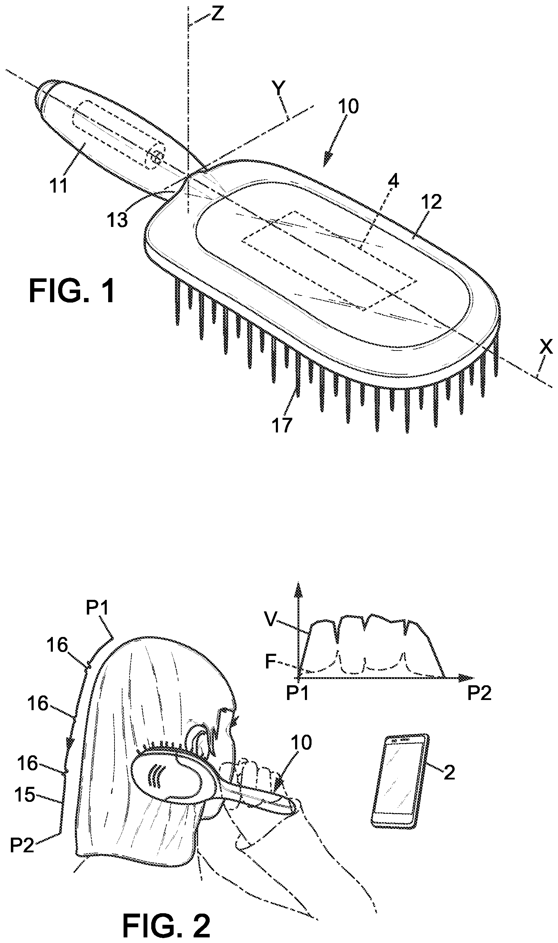

La figure 1 représente un exemple d'ustensile de coiffage, en l'occurrence une brosse à cheveux **10** selon la présente invention. L'ustensile de coiffage pourrait tout aussi bien être un peigne ou un autre instrument de type voisin.

La brosse comprend un manche 11 de préhension (on dit aussi 'poignée') et un corps **12,** lequel est muni sur sa face antérieure **14** de dents **17** ('picots') et/ou de poils, lesquels peuvent s'engager dans la chevelure d'un utilisateur ou d'une utilisatrice. Lorsque ladite brosse se déplace dans la chevelure, une action de coiffage (ou de 'peignage') se produit.

Comme illustré sur la Figure 1, le manche **11** et le corps **12** s'étendent l'un à la suite de l'autre le long d'une direction longitudinale **X ;** la poignée de l'exemple illustré est sensiblement de révolution autour de l'axe X et est reliée au corps **12** au travers d'une zone de transition **13,** qui peut être de section réduite par rapport à la section principale de la poignée. Le corps **12** s'étend principalement dans un plan selon la direction longitudinale **X** et une direction transversale **Y,** tandis que les dents s'étendent selon une troisième direction **Z** nommée radiale.

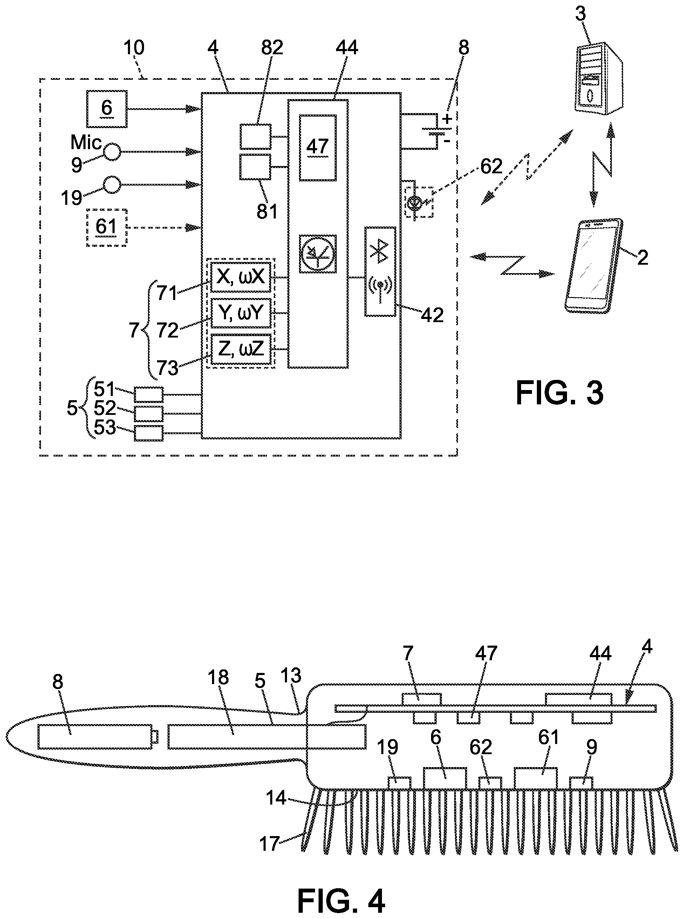

En référence aux figures 2 à 4, la brosse comprend en outre une batterie d'accumulateur électrique **8.** Cette batterie est destinée à alimenter électriquement tous les équipements électriques et électroniques logés dans la brosse qui seront détaillés plus loin. Cette batterie peut être du type rechargeable et peut être rechargée au travers d'une connexion externe ou sans connexion au moyen d'un flux magnétique alternatif (socle de recharge sans contact). Une solution de recharge par flux lumineux n'est pas exclue, par exemple au moyen d'un arrangement à photodiodes, arrangées en surface du corps.

La brosse comprend en outre une unité électronique de commande **4,** s'étendant à l'intérieur du corps sensiblement dans le plan **XY,** dont la constitution et les fonctions seront détaillées plus loin.

La brosse comprend au moins un capteur de mouvement ou accéléromètre **7.** Dans l'exemple illustré, il s'agit d'un capteur six axes, capable de mesurer les accélérations linéaires subies selon les trois directions orthogonales X, Y et Z ainsi que les mouvements de rotation autour des trois directions orthogonales X, Y et Z. Pour la détection des mouvements de rotation, on fait appel à des gyromètres miniatures **71,72,73,** de préférence intégrés dans un package électronique unique (comprenant également les accéléromètres linéaires, par exemple dans un circuit dit 'MEMS') installé directement sur la carte de l'unité de commande.

Grâce aux informations brutes détectées par les accéléromètres et les gyromètres, il est possible de procéder à des calculs d'intégration pour remonter à la détermination des déplacements de la brosse.

Il faut noter cependant que l'accélération engendrée par la pesanteur **g** doit être éliminée, ce qui peut être fait par des opérations de filtrage logiciel ou par l'utilisation d'un inclinomètre multiaxe complémentaire.

Toutefois, le capteur de mouvement pourrait être aussi beaucoup plus simple, par exemple deux axes.

La brosse comprend au moins un moyen de communication sans fil **42,** par exemple sous forme d'une interface Bluetooth™ ou Wifi, agencée sur la carte de l'unité de commande. Cette interface est adaptée pour permettre la transmission de données depuis la brosse vers une entité distante ; la brosse peut aussi recevoir des données via cette interface.

L'entité distante en question est dans l'exemple illustré un appareil mobile de type téléphone intelligent **2** aussi appelé Smartphone ; toutefois l'entité distante pourrait aussi être directement un ordinateur connecté à Internet ou un serveur de données informatiques.

Avantageusement selon l'invention, la brosse peut aussi comprendre au moins un capteur d'effort **5,** qui se présente dans l'exemple illustré comme une tige **18** instrumentée, agencée au moins dans la zone de transition 13 déjà mentionnée plus haut. La tige 18 susmentionnée reprend les efforts entre le manche et le corps.

Dans l'exemple illustré, le capteur d'effort peut être du type jauge de contrainte et est adapté pour mesurer trois composantes **51,52,53** i.e. une torsion autour de l'axe **X,** une flexion autour de l'axe **Y** et une flexion autour de l'axe **Z.** Ledit capteur d'effort mesure par conséquent les efforts principaux exercés entre le manche **11** tenu par l'utilisateur et le corps **12** en engagement avec la chevelure dudit utilisateur, au cours du mouvement de coiffage.

Comme représenté sur la figure 2, un mouvement de coiffage commence en un point de départ **P1**, puis parcourt une ligne généralement courbe **15** jusqu'à un point d'extrémité **P2.** Au cours de ce mouvement, la vitesse de déplacement **V** et les efforts subis **F** évoluent, comme illustré en exemple sur le graphique ; en particulier, au cours des mouvements, il peut y avoir des points ou des zones plus dures **16.** Grâce aux accéléromètres et au capteur d'effort, on détermine la vitesse et la régularité du geste de coiffure, la rotation de la brosse pendant le geste de coiffure, la longueur de la courbe suivie par le geste de coiffure, et le temps mis pour parcourir la dite courbe.

Avantageusement, la brosse peut comprendre en outre au moins une caméra **6** et/ou un capteur optique **61,** agencés sur la face antérieure **14** du corps de la brosse ; leur objectif est par conséquent dirigé vers l'extérieur pour venir recueillir des informations optiques concernant notamment les cheveux et/ou le cuir chevelu.

Une ou plusieurs lampes **62** (ou Leds), travaillant dans le domaine visible ou le domaine infrarouge, sont également disposées sur la face antérieure 14, de manière à pouvoir éclairer la zone de travail lors du mouvement de coiffage.

Le capteur optique **61** peut présenter un niveau de définition plus ou moins élaboré, de quelques centaines de pixels à plusieurs millions de pixels ; il en va de même pour les images captées par la caméra **6.**

Il est entendu que les images peuvent être captées sous différentes conditions lumineuses créées par les lampes **62,** de façon à pouvoir effectuer une radiographie optique des cheveux et/ou du cuir chevelu, notamment pour en tirer des enseignements sur l'état de santé de la chevelure.

De façon avantageuse, on peut prévoir un (ou plusieurs) capteur optique dédié à l'analyse optique des cheveux et au moins un autre capteur optique dédié à l'analyse optique du cuir chevelu.

Il faut noter que les images du cuir chevelu peuvent être reportées sur l'écran d'affichage du Smartphone, de manière à ce que l'utilisateur puisse constater de visu avec un grossissement important l'état de sa chevelure et de son propre cuir chevelu, inaccessible en temps normal ; cette fonction peut être appelée 'fonction webcam capillaire'.

Par ailleurs, la brosse à cheveux peut comprendre en outre un microphone **9,** agencé par exemple au voisinage de la face antérieure **14.** Ce microphone est adapté pour mesurer des frottements et vibrations engendrés par le mouvement de coiffage. L'unité de commande **4** est configurée pour mettre en forme et transmettre des données correspondant aux frottements et vibrations captées par le microphone.

Par ailleurs, la brosse à cheveux peut comprendre en outre un capteur de champ électrique **19,** lui aussi agencé au voisinage de la face antérieure **14** et adapté pour mesurer un champ électrique **E** engendré par des charges électrostatiques dues aux mouvements de coiffage. L'unité de commande **4** est configurée pour mettre en forme et transmettre des données correspondantes au champ électrique mesuré pendant le mouvement de coiffage.

Par ailleurs, la brosse à cheveux peut comprendre en outre un capteur de température **81** et/ou un capteur d'humidité **82,** pour acquérir les caractéristiques d'environnement atmosphérique qui peuvent influer sur les opérations de coiffage et par conséquent peuvent influer sur l'analyse des efforts et des images collectés pendant les mouvements de coiffage.

L'unité de commande **4** comprend un microcontrôleur **44** chargé de gérer les interfaces d'entrée-sortie des capteurs, lampes, et interface de communication, les fonctions principales en mode actif ainsi que les fonctions de réveil et mise en sommeil qui seront décrites plus loin. L'unité de commande **4** est configurée pour pouvoir stocker dans un espace mémoire **47** des données, brutes ou prétraitées, collectées pendant le ou les mouvements de coiffage, préalablement à une transmission ultérieure vers l'entité distante **2.**

Les données collectées pendant les opérations de coiffage sont transmises, avec ou sans traitement préalable, par le Smartphone **2** vers un serveur informatique **3** qui comprend une ou plusieurs applications en charge d'analyser les données en question, de manière à pouvoir formuler des préconisations de soins ou de produits à appliquer à la chevelure considérée.

Les préconisations de soins ou d'application de produits envoyés par le serveur informatique peuvent prendre diverses formes, par exemple en particulier des messages destinés à être affichés sur le Smartphone.

Il faut noter que la remontée des données au travers du Smartphone n'est pas obligatoire, les données pouvant être transmises directement de la brosse à destination du serveur par une liaison de type Wifi.

Il faut également noter qu'il est possible de gérer plusieurs utilisateurs distincts pour une brosse à cheveux donnée. Par ailleurs, chacun des ustensiles de coiffage selon l'invention est identifié unitairement de manière à ce qu'il soit reconnu sans ambiguïté par le Smartphone et/ou le serveur informatique distant.

La batterie électrique peut être de type classique et logée dans le manche comme illustré, mais toutefois la batterie peut prendre une toute autre forme et être logée ailleurs dans la brosse ou dans le peigne. De même, l'unité de commande pourrait être agencée différemment et n'est pas nécessairement logée dans le corps, notamment pour un peigne.

Par ailleurs, l'unité électronique peut gérer une horloge représentant l'heure de la journée ainsi que la date du jour, de manière à pouvoir horodater les opérations de coiffage. La brosse peut recevoir des messages de mise à l'heure de l'horloge et de la date depuis le serveur informatique au travers de l'interface de communication.

Les accéléromètres linéaires sont utilisés pour, d'une part gérer le réveil de l'unité de commande lorsque des mouvements substantiels sont captés par ces accéléromètres, et d'autre part gérer l'endormissement de l'unité de commande après un temps prédéterminé écoulé sans mouvement substantiel capté par les accéléromètres ; on assure ainsi une consommation électrique la plus faible possible, notamment pendant toutes les périodes où la brosse n'est pas utilisée.

Il peut être prévu un seuil d'accélération prédéterminé au-delà duquel l'unité de commande se réveille et en deçà duquel l'unité de commande reste endormie, de manière à pouvoir éviter que l'unité de commande ne se réveille lorsque la brosse est simplement posée dans un véhicule en mouvement comme une automobile, un train, un bateau ou un avion. En revanche, une secousse générée par la manipulation de l'utilisateur réveillera l'unité de commande 4.

S'agissant de l'analyse des données collectées au cours du mouvement de coiffage, notamment les déplacements, les mouvements, les efforts, les informations optiques, le champ électrique, etc, une première étape d'analyse peut être réalisée par le microcontrôleur **44** de l'unité de commande **4** de manière à pouvoir transmettre des données de taille réduite à destination de l'unité distante. Toutefois il n'est pas exclu que l'unité de commande **4** transmette les données sous forme brute pour toute ou partie d'entre elles.

Si les données collectées transitent par un appareil mobile de type Smartphone **2,** cet appareil peut réaliser une deuxième étape d'analyse des données, d'une part pour restituer certaines de ces données à destination de l'utilisateur sur l'écran d'affichage (fonction webcam capillaire) et d'autre part pour pouvoir transmettre des données de plus haut niveau à destination du serveur informatique 3 susmentionné.

Le serveur informatique 3 peut quant à lui procéder à une troisième étape d'analyse des données, et notamment comparer les données reçues à des données de référence stockées dans une base de données relative aux chevelures, cuir chevelu et toute autre information pertinente. Ceci permet d'effectuer un diagnostic à distance par rapport à un ensemble de connaissances capillaires préétablies, et à la connaissance des données antérieures de l'utilisateur en question.

Plus précisément, avantageusement selon l'invention, le serveur informatique 3 fait office de plate-forme centralisée, destinée à collecter un grand nombre d'informations au sujet des opérations de coiffage d'une multitude d'utilisateurs.

En outre, cette plate-forme centralisée peut collecter et stocker les données recueillies relatives à une brosse identifiée particulière, voire même à un utilisateur identifié, et ceci sur une période significative voire une longue période. La plate-forme centralisée peut ainsi analyser les variations sur une longue période notamment concernant les efforts et les mouvements de coiffage d'un utilisateur particulier, ce qui permet de personnaliser, sur la base de données propres à cet utilisateur, le message de préconisations de soins à signifier à cet utilisateur.

Il faut remarquer que la plate-forme peut analyser par apprentissage une grande quantité de données reçues en provenance des utilisateurs des ustensiles de coiffage connectés.

Il faut aussi remarquer que, dans une alternative, le rôle de plate-forme centralisée pourrait être joué par le téléphone de type Smartphone 2.

Dans tous les cas de figure, l'entité distante, à savoir préférentiellement la plate-forme centralisée hébergée dans le serveur 3, fournit, en retour de l'analyse de données, des préconisations comme indiqué plus haut, sous forme de message de type SMS, email ou autre à destination de l'utilisateur.

## Revendications

1. **Système** comprenant un ustensile de coiffage (10) et une entité distante (2,3) pouvant être reliée par une connexion sans fil audit ustensile de coiffage, l'ustensile de coiffage (10) étant formé comme un peigne ou une brosse à cheveux, et comprenant :
- un manche (11) et un corps (12) muni de dents et/ou de poils,
- une batterie d'accumulateur électrique (8),
- au moins un capteur de mouvement ou accéléromètre (7),
- au moins un capteur d'effort (5) adapté pour mesurer un effort de torsion ou de flexion exercé entre le manche (11) tenu par un utilisateur et le corps (12) en engagement avec la chevelure dudit utilisateur,
- une unité électronique (4) de commande, configurée pour acquérir et mettre en forme des signaux fournis par le capteur de mouvement (7) et configurée pour mettre en forme et transmettre des efforts de torsion ou de flexion fournis par le capteur d'effort (5),
- au moins un moyen de communication sans fil (42) adapté pour permettre la transmission de données vers l'entité distante (2,3),
moyennant quoi des informations sur les mouvements impartis à l'ustensile et sur les efforts engendrés par le mouvement de coiffage peuvent être transmis à l'entité distante (2, 3), **caractérisé en ce que** l'entité distante (2,3) est configurée pour formuler des préconisations de soins ou de produits à appliquer à la chevelure considérée et pour fournir ces préconisations à l'utilisateur de l'ustensile de coiffage sous forme de message de type SMS, email ou autre à destination de l'utilisateur.

2. Système selon la revendication 1, dans lequel l'ustensile de coiffage comprend en outre au moins une caméra (6) et/ou un capteur optique (61), adapté pour acquérir des images, notamment des cheveux et/ou du cuir chevelu, l'unité électronique étant configurée pour mettre en forme et transmettre ces images.

3. Système selon l'une des revendications 1 à 2, dans lequel l'ustensile de coiffage comprend en outre au moins un microphone (9), adapté pour mesurer des frottements engendrés par le mouvement de coiffage, l'unité électronique étant configurée pour mettre en forme et transmettre des données correspondantes.

4. Système selon l'une des revendications 1 à 3, dans lequel l'ustensile de coiffage comprend en outre au moins un capteur de champ électrique (19) adapté pour mesurer un champ électrique engendré par des charges électrostatiques dues aux mouvements de coiffage, l'unité électronique étant configurée pour mettre en forme et transmettre des données correspondantes.

5. Système selon l'une des revendications 1 à 4, dans lequel l'ustensile de coiffage comprend en outre au moins un espace mémoire (47) adapté pour permettre le stockage des données collectées pendant le ou les mouvements de coiffage, préalablement à une transmission ultérieure vers l'entité distante.

6. Système selon l'une des revendications 1 à 5, dans lequel l'entité distante (2,3) est formée par un appareil mobile (2), par exemple un Smartphone, configuré pour afficher certaines des données transmises par l'ustensile de coiffage et donner des indications à l'utilisateur à propos de sa chevelure soumise à coiffure.

7. Système selon l'une des revendications 1 à 5, dans lequel l'entité distante (2,3) comprend à la fois un appareil mobile (2) et un serveur informatique (3) pouvant être connecté à l'appareil mobile (2), afin d'échanger des données de synthèse relatives au coiffage.

8. **Procédé** mis en oeuvre dans un système selon la revendication 1 comprenant un ustensile de coiffage (10) et une entité distante (2,3) pouvant être reliée par une connexion sans fil audit ustensile de coiffage, l'ustensile de coiffage (10) comprenant :
- un manche (11) et un corps (12) muni de dents et/ou de poils,
- au moins un capteur de mouvement (7),
- au moins un capteur d'effort (5) adapté pour mesurer un effort de torsion ou de flexion exercé entre le manche (11) tenu par un utilisateur et le corps (12) en engagement avec la chevelure dudit utilisateur,
- une unité électronique (4) de commande, configurée pour acquérir et mettre en forme des signaux fournis par le capteur de mouvement (7) et le capteur d'effort (5),
- au moins un moyen de communication sans fil (42) adapté pour permettre la transmission de données vers l'entité distante (2,3), le procédé comprenant les étapes :
**a**- acquérir des informations relatives au moins aux mouvements de coiffage d'un utilisateur, et relatives aux efforts engendrés par le mouvement de coiffage,
**b**- transmettre des données correspondantes aux dites informations vers l'entité distante,
**e**- recevoir de l'entité distante (2,3) des préconisations de soins ou de produits à appliquer à la chevelure considérée,
**f-** fournir à l'utilisateur de l'ustensile de coiffage des préconisations de soins ou de produits à appliquer à la chevelure considérée, sous forme de message de type SMS, email ou autre à destination de l'utilisateur.

9. **Procédé** selon la revendication 8, dans lequel il est prévu après l'étape b- :
**c**- afficher certaines desdites données correspondantes sur un écran de l'entité distante.

10. **Procédé** selon la revendication 8, dans lequel l'entité distante (2,3) est formée par un appareil mobile (2), par exemple un Smartphone et un serveur informatique (3).

## Patentansprüche

1. System aufweisend ein Frisierwerkzeug (10) und eine Ferneinheit (2, 3), die über eine drahtlose Verbindung mit dem Frisierwerkzeug verbunden werden kann, wobei das Frisierwerkzeug (10) als ein Kamm oder eine Haarbürste gebildet ist und aufweist:
- einen Arm (11) und einen mit Zacken und/oder Borsten versehenen Körper (12),
- eine elektrische Akkumulatorbatterie (8),
- mindestens einen Bewegungs- oder Beschleunigungssensor (7),
- mindestens einen Kraftsensor (5), der eingerichtet ist, eine Torsions- oder Biegekraft zu messen, die zwischen dem von einem Benutzer gehaltenen Arm (11) und dem Körper (12), der mit den Haaren des Benutzers in Eingriff ist, ausgeübt wird,
- eine elektronische Steuereinheit (4), die konfiguriert ist, von dem Bewegungssensor (7) bereitgestellte Signale zu gewinnen und in Form zu bringen, und konfiguriert ist, von dem Kraftsensor (5) bereitgestellte Torsions- oder Biegekräfte (Signale) in Form zu bringen und zu übertragen,
- mindestens eine drahtlose Kommunikationseinrichtung (42), die eingerichtet ist, die Übertragung von Daten zu der Ferneinheit (2, 3) zu erlauben, wodurch Informationen über die dem Werkzeug vermittelten Bewegungen und über die von der Frisierbewegung hervorgerufenen Kräfte zu der Ferneinheit (2, 3) übertragen werden können,
**dadurch gekennzeichnet, dass** die Ferneinheit (2, 3) konfiguriert ist, Empfehlungen für Pflegebehandlungen oder Produkte, die auf das betrachtete Haar anzuwenden sind, zu formulieren und diese Empfehlungen dem Benutzer des Frisierwerkzeugs bereitzustellen im Form einer an den Benutzer gerichteten Nachricht des Typs SMS, Email oder dergleichen.

2. System nach Anspruch 1, in welchem das Frisierwerkzeug ferner mindestens eine Kamera (6) und/oder einen optischen Sensor (61) aufweist, der zum Erfassen von Bildern, insbesondere der Haare und/oder der Kopfhaut eingerichtet ist, wobei die elektronische Einheit konfiguriert ist, diese Bilder in Form zu bringen und zu übertragen.

3. System nach einem der Ansprüche 1 bis 2, in welchem das Frisierwerkzeug ferner mindestens ein Mikrofon (9) aufweist, das eingerichtet ist, die von der Frisierbewegung hervorgerufenen Reibungen zu messen, wobei die elektronische Einheit konfiguriert ist, entsprechende Daten in Form zu bringen und zu übertragen.

4. System nach einem der Ansprüche 1 bis 3, in welchem das Frisierwerkzeug ferner mindestens einen Sensor für elektrisches Feld (19) aufweist, der eingerichtet ist, ein elektrisches Feld zu messen, das von elektrostatischen Aufladungen hervorgerufen wird, die sich aus den Frisierbewegungen ergeben, wobei die elektronische Einheit konfiguriert ist, entsprechende Daten in Form zu bringen und zu übertragen.

5. System nach einem der Ansprüche 1 bis 4, in welchem das Frisierwerkzeug ferner mindestens einen Speicher (47) aufweist, der eingerichtet ist, um das Speichern von Daten, die während der oder den Frisierbewegung(en) gesammelt werden, vor einer späteren Übertragung zu der Ferneinheit zu erlauben.

6. System nach einem der Ansprüche 1 bis 5, in welchem die Ferneinheit (2, 3) aus einem Mobilgerät (2), zum Beispiel einem Smartphone, besteht, das konfiguriert ist, gewisse von dem Frisierwerkzeug übertragene Daten anzuzeigen und dem Benutzer Hinweise zu geben, die seine frisierten Haare betreffen.

7. System nach einem der Ansprüche 1 bis 5, in welchem die Ferneinheit (2, 3) sowohl ein Mobilgerät (2) als auch einen Computerserver (3) aufweist, der mit dem Mobilgerät (2) verbunden werden kann, um mit dem Frisieren verwandte Synthesedaten auszutauschen.

8. Verfahren, das in einem System nach Anspruch 1 durchgeführt wird, das ein Frisierwerkzeug (10) und eine Ferneinheit (2, 3), die über eine drahtlose Verbindung mit dem Frisierwerkzeug verbunden werden kann, aufweist, wobei das Frisierwerkzeug (10) aufweist:
- einen Arm (11) und einen mit Zacken und/oder Borsten versehenen Körper (12),
- mindestens einen Bewegungssensor (7),
- mindestens einen Kraftsensor (5), der eingerichtet ist, eine Torsions- oder Biegekraft zu messen, die zwischen dem von einem Benutzer gehaltenen Arm (11) und dem Körper (12), der mit den Haaren des Benutzers in Eingriff ist, ausgeübt wird,
- eine elektronische Steuereinheit (4), die konfiguriert ist, von dem Bewegungssensor (7) und dem Kraftsensor (5) bereitgestellte Signale zu gewinnen und in Form zu bringen,
- mindestens eine drahtlose Kommunikationseinrichtung (42) die eingerichtet ist, die Übertragung von Daten zu der Ferneinheit (2, 3) zu erlauben,
wobei das Verfahren die folgenden Schritte aufweist:
a- Gewinnen von Informationen, die mindestens die Frisierbewegungen eines Benutzers betreffen und die von der Frisierbewegung hervorgerufenen Kräfte betreffen,
b- Senden von Daten, die den Informationen entsprechen, zu der Ferneinheit,
e- Empfangen von Empfehlungen für Pflegebehandlungen und Produkte, die auf das betrachtete Haar anzuwenden sind, aus der Ferneinheit (2, 3),
f- Versorgen des Benutzers des Frisierwerkzeugs mit Empfehlungen für Pflegebehandlungen und Produkte, die auf das betrachtete Haar anzuwenden sind, in Form einer an den Benutzer gerichteten Nachricht des Typs SMS, Email oder dergleichen.

9. Verfahren nach Anspruch 8, in welchem nach dem Schritt b- vorgesehen ist:
c- Anzeigen von gewissen der entsprechenden Daten auf einem Bildschirm der Ferneinheit.

10. Verfahren nach Anspruch 8, in welchem die Ferneinheit (2, 3) aus einem Mobilgerät (2), zum Beispiel einem Smartphone, und einem Computerserver (3) besteht.

## Claims

1. System comprising a hairstyling tool (10) and a remote entity (2,3) which can be connected to said hairstyling tool by a wireless connection, the hairstyling tool(10) being a comb or a hairbrush, and comprising:
- a handle (11) and a body (12), provided with teeth and/or hairs,
- a battery for storing electricity (8),
- at least one movement sensor or accelerometer (7),
- at least one force sensor (5) suitable for measuring a torsional or bending stress exerted between the handle (11) held by a user and the body (12) engaged with the hair of a user,
- an electronic control unit (4), configured to capture and format signals provided by the movement sensor (7), and configured to format torsional or bending stress exerted provided by the force sensor (5),
- at least one wireless communication means (42) suitable for data transmission to the remote entity (2,3), whereby information on the movements executed by the hairstyling tool can be transmitted to the remote entity (2,3), **characterized in that** the entity (2,3) is configured to state recommendations for hair care or products to be applied to the hair concerned, and to provide these recommendations to the user of the hairstyling tool as a short message, email or other to the user.

2. System according to claim 1, wherein the hairstyling tool further comprises at least one camera (6) and/or optical sensor (61) suitable for capturing images of the hair and/or scalp, the electronic unit being configured to format and transmit these images.

3. System according to any of the claims 1 to 2, wherein the hairstyling tool further comprises at least one microphone suitable for measuring the friction generated by the hairstyling movement, the electronic unit being configured to format and transmit the corresponding data.

4. The System according to any of the claims 1 to 3, wherein the hairstyling tool further comprises at least one electric field sensor (19) suitable for measuring an electric field generated by the electrostatic charges resulting from hairstyling movements, the electronic unit being configured to format and transmit the corresponding data.

5. System according to of the claims 1 to 4, wherein the hairstyling tool further comprises at least one memory space (47) suitable for storing data collected during the hairstyling movement or movements, prior to subsequent transmission to the remote entity.

6. System according to of the claims 1 to 5, wherein the remote entity (2,3) is formed of a mobile device (2), for example a smartphone, configured to display some of the data transmitted by the hairstyling tool and provide information to the user about the hair being styled.

7. System according to of the claims 1 to 6, wherein the remote entity (2,3) comprises both a mobile device (2) and a computer server (3) that can connect to the mobile device (2) in order to exchange summary data concerning the hairstyling.

8. **Method** implemented in a system according to claim 1 comprising a hairstyling tool (10) and a remote entity (2,3) which can be connected to said hairstyling tool by a wireless connection, the hairstyling tool(10) comprising :
- a handle (11) and a body (12), provided with teeth and/or hairs,
- at least one movement sensor (7),
- at least one force sensor (5) suitable for measuring a torsional or bending stress exerted between the handle (11) held by a user and the body (12) engaged with the hair of a user,
- an electronic control unit (4), configured to capture and format signals provided by the movement sensor (7), and configured to format torsional or bending stress exerted provided by the force sensor (5),
- at least one wireless communication means (42) suitable for data transmission to the remote entity (2,3),
the method comprising the steps of:
**a-** capturing information relating to at least the hairstyling movements of a user, and relating to forces generated by the hairstyling movement,
**b-** transmitting the data corresponding to such information to the remote entity (2,3),
**e-** receiving back from the server recommendations for hair care or products to be applied to the hair concerned, and
displaying them on the mobile device (2).
**f-** providing to the user of the hairstyling tool hair care or products recommendations as a short message, email or other to the user.

9. Method according to claim 8, further comprising, after step b-,
**c-** displaying some of said corresponding data on a screen of the remote entity.

10. Method according to claim 8, wherein the remote entity (2,3) is formed as a mobile device (2), for example a smartphone, and a computer server (3).
